(19) Europäisches Patentamt
European Patent Office
Office européen des brevets

(11) Veröffentlichungsnummer: **0 113 812**
**B1**

(12) # EUROPÄISCHE PATENTSCHRIFT

(45) Veröffentlichungstag der Patentschrift: **26.10.88**

(51) Int. Cl.⁴: **C 07 J 1/00**, C 07 J 63/00, A 61 K 49/02

(21) Anmeldenummer: **83109753.0**

(22) Anmeldetag: **29.09.83**

(54) **Verfahren zur Herstellung von 17alpha-Bromethinyl- und 17alpha-Jodethinyl-17beta-hydroxysteroiden und neue 17alpha-Bromethinyl- und 17alpha-Jodethinyl-17beta-hydroxysteroide.**

(30) Priorität: **16.11.82 DE 3242892**

(43) Veröffentlichungstag der Anmeldung: **25.07.84 Patentblatt 84/30**

(45) Bekanntmachung des Hinweises auf die Patenterteilung: **26.10.88 Patentblatt 88/43**

(84) Benannte Vertragsstaaten: **AT BE CH DE FR GB IT LI LU NL SE**

(56) Entgegenhaltungen:
**GB - A - 1 043 675**
**US - A - 3 100 204**

**THE JOURNAL OF NUCLEAR MEDICINE, Band 21, Nr. 2, Februar 1980, Seiten 142-146, New York, USA, J. KRIJN MAZAITIS et al.: "Radioiodinated estrogen derivatives"**
**CHEMICAL ABSTRACTS, Band 96, Nr. 15, 12. April 1982, Seite 729, Nr. 123080r, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Hofmeister, Helmut, Dr., Weislingenstrasse 4, D-1000 Berlin 28 (DE)**
Erfinder: **Schulze, Paul Eberhard, Dr., Endestrasse 30, D-1000 Berlin 39 (DE)**
Erfinder: **Annen, Klaus, Dr., Seegefelder Strasse 194, D-1000 Berlin 20 (DE)**
Erfinder: **Laurent, Henry, Dr., Glambecker Weg 21, D-1000 Berlin 28 (DE)**
Erfinder: **Wiechert, Rudolf, Prof., Petzower Strasse 8A, D-1000 Berlin 39 (DE)**

EP 0 113 812 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung von Brom und Jod unmarkierten und radioaktiv markierten 17α-Bromethinyl- und 17α-Jodethinyl-17β-hydroxysteroiden der allgemeinen Formeln I und II

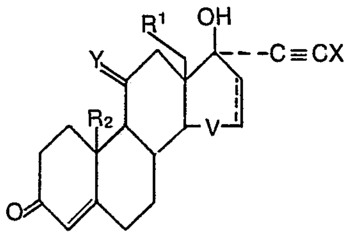

(I),

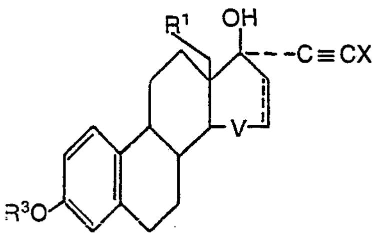

(II),

worin

..... eine Einfachbindung oder Doppelbindung ist,

V eine Kohlenstoff-Kohlenstoffbindung oder eine Methylengruppe darstellt,

X für ein Brom oder Jod steht,

Y zwei Wasserstoffatome oder eine Methylengruppe darstellt,

$R^1$ und $R^2$ ein Wasserstoffatom oder eine Methylgruppe bedeutet und

$R^3$ eine Alkylgrupppe mit bis zu 4 Kohlenstoffatomen oder ein Wasserstoffatom darstellt aus entsprechenden 17α-Ethinyl-17β-hydroxysteroiden, welches sich dadurch auszeichnet, dass man das Ausgangssteroid in einem inerten Lösungsmittel mit einem Bromierungsmittel bzw. Jodierungsmittel in Gegenwart eines Silbersalzes behandelt sowie neue Verbindungen aus der Reihe von 17α-Bromethinyl- oder 17α-Jodethinyl-17β-hydroxysteroiden der allgemeinen Formel Ia

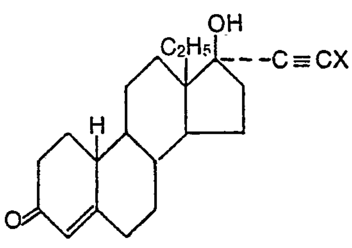

(Ia),

worin

X die vorstehend genannte Bedeutung besitzt.

Die erfindungsgemäss herstellbaren Verbindungen sind bekannte und neue Substanzen mit einem Wirkungsprofil wie die entsprechenden Verbindungen ohne Halogen. So haben die Verbindungen der vorstehend definierten Formel I eine gestagene Wirksamkeit, wie beispielsweise Levonorgestrel. Die Verbindungen der allgemeinen Formel II haben ebenso wie beispielsweise Ethinylöstradiol eine östrogene Wirksamkeit. Sie dienen zum Teil auch als Diagnostika in der Medizin, wie z.B. das 17α-[*Br]-Brom oder 17α-[*J]-Jod-ethinyl-estradiol. [J. Nucl. Med. 21, 142, (1989)].

Bekanntermassen lassen sich 17α-Halogenethinyl-17β-hydroxysteroide der Androstan- und Östranreihe dadurch herstellen, dass man die 3- und 17β-ständigen Sauerstoffunktionen eines 4(5)-ungesättigten Steroids durch Schutzgruppen schützt, die so erhaltene Verbindung unter Rückfluss mit Kalium-tert.-butylat und einem Halogenierungsmittel wie N-Bromsuccinimid in einem tertiären Alkohol umsetzt und die Schutzgruppen wieder abspaltet (DE-A-1 242 607 und USA-3 100 204).

Ein anderes Verfahren verwendet als Bromierungsmittel Trifluorbrommethan, das in flüssigem Ammoniak mit dem Lithiumsalz des 17α-Ethinylsteroids zur Reaktion gebracht wird. Auch hierbei müssen die Sauerstoffunktionen am C-3 und die 17β-Hydroxygruppe geschützt sein (Tetrahedron 23, 1967, 4111).

Ferner ist ein Verfahren zur Herstellung von 17α-Bromethinyl-1,3,5(10)-estratrien-3,17β-diol bekannt, bei dem man 3-Benzoyloxy-17α-ethinyl-1,3,5(10)-estratrien-17β-ol mit Chloramin T und Natriumbromid umsetzt und anschliessend die 3-Benzoylgruppe abspaltet (C.A. 96, 1982, Nr. 123080r).

Letztlich ist es möglich, 17α-Bromethinylsteroide aus den entsprechenden 17-Oxosteroiden durch Umsetzung mit Dibromethylen herzustellen (GB-A-1 043 675).

Von den 17α-Jodethinylsteroiden sind bisher nur 17α-Jodethinyl-1,3,5(10)-estratrien-3,17β-diol und dessen 11β-Methoxy-Derivat bekannt, die aus 3-Benzoyloxy-17α-ethinyl-1,3,5(10)-estratrien-17β-ol bzw. dessen 11β-Methoxy-Derivat durch Reaktion mit Morpholin und Jod oder mit Chloramin-T und Natriumjodid und anschliessender Verseifung der 3-Benzoyloxy-Gruppe erhalten werden [J. Nucl. Med. 21, 142 (1980)].

Aufgabe der vorliegenden Erfindung war es somit, ein Verfahren zur Herstellung von 17α-Bromethinyl- oder 17α-Jodethinyl-17β-hydroxysteroiden aus den entsprechenden 17α-Ethinylcarbinolen bereitzustellen, welches ohne Schutz der 17β-Hydroxygruppe und einer gegebenenfalls vorhandenen 3-Ketogruppe die Halogenierung der 17α-Ethinylgruppe ermöglicht.

Die erfindungsgemässe Aufgabe wurde so gelöst, dass man zu dem Reaktonsgemisch aus Bromierungs- bzw. Jodierungsmittel und Ausgangssteroid eine katalytische Menge Silbersalz gibt. Die Reaktion wird in einem protischen oder aprotischen Lösungsmittel oder in Lösungsmittelgemischen durchgeführt.

Das Wesen der Erfindung liegt in der Verwendung eines Silbersalzes als Katalysator, der in einer Menge von 1/1000 bis 1, vorzugsweise 1/100 bis 1/10 Moläquivalenten bezogen auf das Ausgangssteroid eingesetzt wird. Geeignete Silbersalze sind beispielsweise Silbernitrat, Silberperchlorat, Silberacetat, Silbertrifluoracetat, Silberfluorid oder Silbersulfat.

Geeignete Bromierungs- bzw. Jodierungsmittel sind Bromkationen oder Jodkationen abgebende Agenzien.

Zur Bromierung eignen sich N-Bromsuccinimid, N-Brom-acetamid, N-Bromphthalimid, N-Brom-p-toluolsulfamid, N-Brom-p-toluolsulfimid, N-Bromcaprolactam, Natriumhypobromid und 1,3-Dibrom-5,5-dimethyl-hydantoin. Besonders geeignet ist N-Bromsuccinimid.

Als Jodierungsmittel dient insbesondere N-Jodsuccinimid.

Das Halogenierungsmittel wird in einer äquimolaren Menge bezogen auf das Ausgangssteroid eingesetzt, wobei jedoch ein Überschuss auch möglich ist.

Als Lösungsmittel lassen sich alle Lösungsmittel verwenden, die sich gegenüber dem Halogenierungsmittel und Silbersalz inert verhalten.

Insbesondere sind Lösungsmittel wie Ketone, z.B. Aceton, Cyclohexanon, Methylethylketon und Methylisobutylketon, cyclische Ether, z.B. Tetrahydrofuran und Dioxan, aliphatische Polyether wie Ethylenglykoldimethylether und Diethylenglykoldimethylether, aromatische Kohlenwasserstoffe wie Benzol oder Toluol, aliphatische Alkohole wie Methanol, Ethanol und Propanol und Lösungsmittel wie Dimethylformamid, Dimethylacetamid, Hexamethylphosphortriamid, N-Methylpyrrolidon wie auch Gemische aus mit Wasser mischbaren Lösungsmitteln und Wasser geeignet.

Die Lösungsmittel können einzeln, soweit sie das Ausgangssteroid in Lösung bringen oder suspendieren, oder auch als Mischung untereinander eingesetzt werden.

Die erfindungsgemässe Reaktion verläuft im Temperaturbereich von 0 bis 50 °C und wird vorzugsweise bei Raumtemperatur durchgeführt. Sie ist in Abhängigkeit vom verwendeten Lösungsmittel innerhalb von 10 Minuten bis 20 Stunden beendet, was z.B. durch Dünnschichtchromatographie festzustellen ist.

Nach beendeter Reaktion wird das Reaktionsgemisch in üblicher Weise, z.B. durch Fällung, Waschen, Extraktion, Umkristallisation und/oder Säulenchromatographie aufgearbeitet.

Der Verlauf der erfindungsgemässen Reaktion war unerwartet und deshalb überraschend, da eine Reaktion am 17α-Ethinylcarbinol-System mit unterbromiger Säure, hergestellt aus N-Bromsuccinimid oder N-Bromacetamid mit tert.-Butanol/Wasser, nur dann stattfindet, wenn die 17β-Hydroxygruppe verestert ist. Im Gegensatz zur erfindungsgemässen silbersalzkatalysierten Methode wird hierbei kein 17α-Bromethinyl-Steroid gebildet, sondern ein 21-Dibromketon (Salomon et al., Helv. Chim. Acta 30 (1947) 1616).

Des weiteren wurde Folgendes festgestellt: Gibt man zu einer Suspension von 17α-Ethinyl-17β-hydroxy-4-androsten-3-on in Aceton/Wasser N-Chlorsuccinimid und eine katalytische Menge Silbersalz, so findet überraschenderweise keine Reaktion zur entsprechenden 17α-Chlorethinyl-Verbindung statt. So war es durchaus überraschend, dass unter den gleichen Reaktionsbedingungen mit N-Brom- bzw. N-Jodsuccinimid das 17α-Bromethinyl- bzw. 17α-Jodethinyl-4-androsten-3-on in hoher Ausbeute erhalten wurde.

Das erfindungsgemässe Verfahren zur Herstellung von 17α-Brom- und 17α-Jodethinyl-Steroiden aus den entsprechenden 17α-Ethinyl-17β-hydroxy-Verbindungen hat gegenüber den bekannten Methoden den Vorteil, dass es ein Einstufenverfahren ist und die Ausbeuten in 17α-Halogenethinyl-Steroiden in den meisten Fällen sehr hoch sind.

17α-Brom- und 17α-Jodethinyl-Steroide können Ausgangsprodukte für die Herstellung der entsprechenden radioaktiv markierten 17α-Halogenethinyl-Verbindungen sein. So lassen sich nach einer radiochemisch neuen Methode aus 17α-Jod- bzw. 17α-Bromethinyl-Steroiden durch Austauschreaktion mit radioaktivem Natrium[*J]-jodid in Aceton 17α-[*J]-Jodethinyl-Verbindungen herstellen. Analog bilden sich aus 17α-Jod- und 17α-Bromethinyl-Steroiden mit radioaktivem Natrium[*Br]-bromid unter Zusatz von Kupfersulfat die entsprechenden 17α-[*Br]-Bromethinyle, die auch nach bekannter Reaktion (J. Label. Comp. and Radiopharmaceuticals 18, 1033 (1980)) aus Jodethinyl-Steroiden durch Umsetzung mit Natrium[*Br]-bromid und Chloramin-T hergestellt werden können. Die radioisotope Markierung mit Brom- bzw. Jodisotopen ist auch über radioaktives Brom- bzw. Jodsuccinimid möglich, indem man 17α-Ethinyl-Steroide mit radioaktivem Halogensuccinimid unter Zusatz katalytischer Mengen eines Silbersalzes in z.B. Aceton/Wasser umsetzt.

Markierte 17α-Halogenethinyl-Steroide sind in der medizinischen Diagnostik von Interesse. So werden 17α-[$^{125}$J]-Jodethinylestradiol (J. Nucl. Med. 21, 142 (1980)) oder 17α-[$^{77}$Br]-Bromethinylestradiol (J. Label Comp. and Radiopharmaceuticals 18, 1033 (1980)) zum Auffinden von Brusttumoren eingesetzt. Die neuen 17α-Bromethinyl- und 17α-Jodethinyl-17β-hydroxysteroide der allgemeinen Formeln I und II können in diagnostischen Mitteln in entsprechender Weise Anwendung finden.

Die nachfolgenden Beispiele sollen das erfindungsgemässe Verfahren und die Anwendung der Verfahrensprodukte erläutern.

Beispiel 1

Eine Suspension von 2,0 g 17α-Ethinyl-17β-hydroxy-18-methyl-4-estren-3-on in 40 ml Aceton und 6 ml Wasser wird bei Raumtemperatur mit 1,4 g N-Bromsuccinimid und 60 mg Silbernitrat versetzt. Nach 30 Minuten gibt man das Reaktionsgemisch in Eis/Wasser. Das ausgefallene Produkt wird abgesaugt, mit Wasser gewaschen, mit Essigester gelöst undd über Natriumsulfat getrocknet. Nach Umkristallisation des Rohproduktes aus Aceton/Hexan werden 2,0 g 17α-Bromethinyl-17β-hydroxy-18-methyl-4-estren-3-on erhalten.

Schmelzpunkt: 206,4 °C – Ausbeute 80% der Theorie.

Beispiel 2

Zu 1,5 g 17α-Ethinyl-3-methoxy-D-homo-1,3,5(10)-estratrien-17aβ-ol in 30 ml Aceton und

4 ml Wasser werden bei Raumtemperatur 1,5 g 1,3-Dibrom-5,5-dimethylhydantoin und 50 mg Silbernitrat gegeben. Nach 30 Minuten wird das Reaktionsgemisch in Eis/Wasser eingerührt. Das ausgefallene Produkt wird abgesaugt, mit Wasser gewaschen und in Essigester gelöst. Nach Chromatographie des Rohproduktes an Kieselgel mit Aceton/Hexan werden 1,3 g 17aα-Bromethinyl-3-methoxy-D-homo-1,3,5(10)-estratrien-17aβ-ol als Schaum isoliert.

Ausbeute 70% der Theorie.

Beispiel 3

Wie im Beispiel 1 beschrieben, aber unter Verwendung von Toluol-Aceton (85:15) als Lösungsmittel wird 17α-Ethinyl-17β-hydroxy-18-methyl-4-estren-3-on bei Raumtemperatur (Reaktionszeit 5 Stunden) umgesetzt und zu 17α-Bromethinyl-17β-hydroxy-18-methyl-4-estren-3-on aufbereitet.

Schmelzpunkt: 203,5 °C – Ausbeute 64% der Theorie.

Beispiel 4

Wie im Beispiel 1 beschrieben, aber unter Verwendung von 1,3-Dibrom-5,5-dimethylhydantoin als Bromierungsmittel wird 17α-Ethinyl-17β-hydroxy-18-methyl-4-estren-3-on bei Raumtemperatur (Reaktionszeit 1 Stunde) umgesetzt und zu 17α-Bromethinyl-17β-hydroxy-18-methyl-4-estren-3-on aufbereitet.

Schmelzpunkt: 204,3 °C – Ausbeute 72% der Theorie.

Beispiel 5

Wie im Beispiel 1 beschrieben, aber unter Verwendung von Ethanol als Lösungsmittel wird 17α-Ethinyl-17β-hydroxy-4-estren-3-on bei Raumtemperatur umgesetzt (Reaktionszeit 30 Minuten) und zu 17α-Bromethinyl-17β-hydroxy-4-estren-3-on aufbereitet.

Schmelzpunkt: 182,5 °C – Ausbeute 64% der Theorie.

Beispiel 6

Wie in Beispiel 1 beschrieben, aber unter Verwendung von 1-Methyl-2-pyrrolidon als Lösungsmittel und Silber-trifluoracetat als Katalysator wird 17α-Ethinyl-17β-hydroxy-4-estren-3-on bei Raumtemperatur umgesetzt (Reaktionszeit 15 Minuten) und zu 17α-Bromethinyl-17β-hydroxy-4-estren-3-on aufbereitet.

Schmelzpunkt: 184,6 °C – Ausbeute 80% der Theorie.

Beispiel 7

Wie in Beispiel 1 beschrieben, aber unter Verwendung von Aceton als Lösungsmittel und Silberacetat als Katalysator wird 17α-Ethinyl-17β-hydroxy-4-androsten-3-on bei Raumtemperatur umgesetzt (Reaktionszeit 45 Minuten) und zu 17α-Bromethinyl-17β-hydroxy-4-androsten-3-on aufbereitet.

Schmelzpunkt: 183,7 °C – Ausbeute 88% der Theorie.

Beispiel 8

Wie in Beispiel 1 beschrieben, aber unter Verwendung von N-Bromacetamid als Bromierungsmittel wird 17α-Ethinyl-17β-hydroxy-4-androsten-3-on bei 30 °C umgesetzt (Reaktionsdauer 20 Stunden) und zu 17α-Bromethinyl-17β-hydroxy-4-androsten-3-on aufbereitet.

Schmelzpunkt: 181,5 °C – Ausbeute 45% der Theorie.

Beispiel 9

Wie in Beispiel 1 beschrieben, aber unter Verwendung von Tetrahydrofuran als Lösungsmittel wird 17α-Ethinyl-17β-hydroxy-18-methyl-11-methylen-4-estren-3-on bei 15 °C umgesetzt (Reaktionszeit 30 Minuten) und zu 17α-Bromethinyl-17β-hydroxy-18-methyl-11-methylen-4-estren-3-on aufbereitet.

Schmelzpunkt: 203,8 °C – Ausbeute 92% der Theorie.

Beispiel 10

Wie in Beispiel 1 beschrieben, aber unter Verwendung von 1,2-Dimethoxyethan als Lösungsmittel wird 17α-Ethinyl-18-methyl-11-methylen-4-estren-17β-ol bei 10 °C umgesetzt, und zu 17α-Bromethinyl-18-methyl-11-methylen-4-estren-17β-ol aufbereitet.

Schmelzpunkt: 168,8 °C – Ausbeute 51% der Theorie.

Beispiel 11

Wie in Beispiel 1 beschrieben, aber unter Verwendung von Silberacetat als Katalysator wird 17α-Ethinyl-17β-hydroxy-18-methyl-4,15-estradien-3-on bei Raumtemperatur umgesetzt (Reaktionsdauer 20 Minuten) und zu 17α-Bromethinyl-17β-hydroxy-18-methyl-4,15-estradien-3-on aufbereitet.

Zersetzungspunkt 110 °C – Ausbeute 50% der Theorie.

Beispiel 12

Wie in Beispiel 1 beschrieben, aber unter Verwendung von 2-Butanon als Lösungsmittel wird 17aα-Ethinyl-3-methoxy-1,3,5(10)-estratrien-17β-ol bei Raumtemperatur umgesetzt (Reaktionsdauer eine Stunde) und zu 17aα-Bromethinyl-3-methoxy-1,3,5(10)-estratrien-17β-ol aufbereitet.

Schmelzpunkt: 172,6 °C – Ausbeute 84% der Theorie.

Beispiel 13

Wie in Beispiel 1 beschrieben, aber unter Verwendung von Dimethylformamid-Wasser (85:15) als Lösungsmittel wird 17α-Ethinyl-1,3,5(10)-estratrien-3,17β-diol bei Raumtemperatur umgesetzt (Reaktionsdauer 4 Stunden) und zu 17α-Bromethinyl-1,3,5(10)-estratrien-3,17β-diol aufbereitet.

Schmelzpunkt 169,6 °C – Ausbeute 46% der Theorie.

Beispiel 14

Zu einer Lösung von 1,0 g 17α-Ethinyl-17β-hydroxy-4-estren-3-on in 20 ml Aceton und 3 ml

Wasser gibt man bei Raumtemperatur 900 mg N-Jodsuccinimid und 50 mg Silbernitrat. Nach 30 Minuten wird das Reaktionsgemisch in Eis/Wasser eingerührt. Das ausgefallene Produkt wird abgesaugt, mit Wasser gewaschen, in Essigester gelöst und über Natriumsulfat getrocknet. Nach Umkristallisation aus Aceton/Hexan erhält man 1,1 g 17β-Hydroxy-17α-jodethinyl-4-östren-3-on.

Zersetzungspunkt 168 °C – Ausbeute 77% der Theorie.

Beispiel 15

Zu 800 mg 17aα-Ethinyl-3-methoxy-D-homo-1,3,5(10)-estratrien-17aα-ol in 20 ml Aceton und 3 ml Wasser gibt man bei Raumtemperatur 610 mg N-Jodsuccinimid und 50 mg Silbernitrat. Nach 20 Minuten wird das Reaktionsgemisch in Eis/Wasser eingerührt. Das ausgefallene Produkt wird abgesaugt, mit Wasser gewaschen und in Essigester gelöst. Nach Chromatographieren des Rohproduktes an Kieselgel mit Aceton/Hexan erhält man 650 mg 17aα-Jodethinyl-3-methoxy-D-homo-1,3,5(10)-estratrien-17aβ-ol als Schaum.

Ausbeute 59% der Theorie.

Beispiel 16

Wie in Beispiel 14, aber unter Verwendung von Aceton als Lösungsmittel und Silber-trifluoracetat als Katalysator wird 17α-Ethinyl-17β-hydroxy-4-androsten-3-on bei 15 °C umgesetzt (Reaktionsdauer 30 Minuten) und zu 17β-Hydroxy-17α-jodethinyl-4-androsten-3-on aufbereitet.

Zersetzungspunkt 164 °C – Ausbeute 79% der Theorie.

Beispiel 17

Wie in Beispiel 14, aber unter Verwendung von Tetrahydrofuran als Lösungsmittel wird 17α-Ethinyl-17β-hydroxy-18-methyl-4-estren-3-on bei 10 °C umgesetzt (Reaktionsdauer 45 Minuten) und zu 17β-Hydroxy-17α-jodethinyl-18-methyl-4-estren-3-on aufbereitet.

Zersetzungspunkt 157 °C – Ausbeute 86% der Theorie.

Beispiel 18

Wie in Beispiel 14, aber unter Verwendung von 1,2-Dimethoxyethan als Lösungsmittel und Silberacetat als Katalysator wird 17α-Ethinyl-17β-hydroxy-18-methyl-11-methylen-4-estren-3-on bei 15 °C umgesetzt (Reaktionsdauer 30 Minuten) und zu 17β-Hydroxy-17α-jodethinyl-18-methyl-11-methylen-4-estren-3-on aufbereitet.

Zersetzungspunkt 180 °C – Ausbeute 56% der Theorie.

Beispiel 19

Wie in Beispiel 14, aber unter Verwendung von Dioxan als Lösungsmittel wird 17α-Ethinyl-3-methoxy-1,3,5(10)-estratrien-17β-ol bei 20 °C umgesetzt (Reaktionsdauer 1¾ Stunde) und zu 17α-Jodethinyl-3-methoxy-1,3,5(10))-estratrien-17β-ol aufbereitet.

Schmelzpunkt: 145 °C – Ausbeute 86% der Theorie.

Beispiel 20

Wie in Beispiel 14 wird 17α-Ethinyl-17β-hydroxy-18-methyl-4,15-estradien-3-on bei Raumtemperatur umgesetzt (Reaktionsdauer 20 Minuten) und zu 17β-Hydroxy-17α-jodethinyl-18-methyl-4,15-estradien-3-on aufbereitet.

Zersetzungspunkt 167 °C – Ausbeute 64% der Theorie.

Beispiel 21

9 mg (0,14 mMol) N-Jodsuccinimid werden mit 1 ml Aceton suspendiert und in Gegenwart von 37 MBq (1 mCi) $Na^{125}J$ (trägerfrei) unter Rückfluss erhitzt. (Im DC-System wird der Fortgang und die Beendigung des Austauschs zwischen radioaktivem Jodid in der Lösung und dem inaktiven Jod im Molekül überprüft.)

Man fügt nach Abkühlen 0,15 Wasser und 10 mg (32 μMol) 17α-Ethinyl-17β-hydroxy-18-methyl-4-östren-3-on zu, gibt dann 0,6 mg (100 μMol) Silbernitrat zu.

Mittels DC-System (Ether/Chloroform 8:2) verfolgt man den Reaktionsablauf und bricht die Reaktion nach ca. 1 Stunde unter Rühren bei Zimmertemperatur ab.

Die Lösung wird mit Wasser verdünnt und mit Essigester extrahiert. Die Essigester-Phase wird mit Wasser neutral gewaschen und eingeengt. Die Rohsubstanz wird über eine Niederdrucksäule chromatographisch gereinigt. Als Fliessmittel verwendet man Hexan/Essigester (Essigester 0–30%). Man erhält 4,07 MBq (110 μCi) 17β-Hydroxy-17α-[$^{125}J$]-jodethinyl-18-methyl-4-östren-3-on mit einer spezifischen Aktivität von ca. 260 MBq/mMol (7 mCi/mMol).

Beispiel 22

5 μg (0,011 μMol) 17β-Hydroxy-17α-jodethinyl-18-methyl-4-östren-3-on werden in 0,25 ml Aceton (p.a.) gelöst und unter Schutzgas mit 370 MBq Natrium[$^{131}J$]-jodid höchster spezifischer Aktivität eine Stunde unter Rückfluss erhitzt.

Nach Abkühlen bis auf Eisbadtemperatur setzt man 0,25 ml Methanol, 0,1 ml Wasser und 1 μg Natriumjodid zu. Zum Entfernen der Ionen zieht man die Lösung durch einen Mischbett-Ionenaustauscher. Man erhält ca. 4 μg 17β-Hydroxy-17α-[$^{131}J$]-jodethinyl-18-methyl-4-östren-3-on bei einer ca. 70%igen radiochemischen Ausbeute mit einer spezifischen Aktivität von ~259 MBq/4 μg (28,4 GBq/μMol). Die chemische und radiochemische Identität der Verbindung wir durch Co-Chromatographie auf DC-Platten und analoge Retentionszeiten in der HPLC bestätigt. Für die diagnostische bzw. therapeutische Anwendung engt man die Lösung unter Vakuum ein, löst in Ethanol/Propylenglykol und schliesst eine Steril-Filtration an.

Auf analoge Weise erhält man die $^{123}J$-, $^{125}J$- und $^{131}J$-Verbindung.

Beispiel 23

5 μ (0,013 μMol) 17α-Bromethinyl-17β-hydroxy-18-methyl-4-östren-3-on werden in 0,25 ml Aceton (p.a.) gelöst und unter Schutzgas in Gegenwart von 370 MBq Natrium[$^{125}J$]-jodid (trägerfrei) und

1µg Kupfersulfat 3 Stunden unter Rückfluss erhitzt.

Nach Abkühlen auf Eisbadtemperatur setzt man 0,25 ml Methanol, 0,1 ml Wasser und 1 g Natriumjodid zu. Zum Entfernen der Ionen zieht man die Lösung durch einen Mischbett-Ionenaustauscher. Man erhält 2,3 µg 17β-Hydroxy-17α-[$^{125}$J]-jodethinyl-18-methyl-4-östren-3-on mit einer spezifischen Aktivität, die der des eingesetzten Na[$^{125}$J]-jodid entspricht. Sie liegt somit in der Grössenordnung von 80,29 GBq/µMol. Das erhaltene, mit $^{125}$J trägerfrei markierte 17β-Hydroxy-17α-jodethinyl-18-methyl-4-östren-3-on wird vom Ausgangsprodukt durch Chromatographie an Silicagel auf einer Niederdrucksäule im System Hexan/Aceton (Aceton 10--- 30%) abgetrennt.

Nach Einengen, Aufnehmen in Ethanol/Propylenglykol, erhält man ein für diagnostische Zwekke geeignetes Produkt.

Beispiel 24

50 µg (0,13 µMol) 17β-Hydroxy-17α-jodethinyl-18-methyl-4-östren-3-on werden in 50 µl Tetrahydrofuran, enthaltend 1 µl 0,5 n Salzsäure, gelöst und zu 370 MBq Natrium[$^{82}$Br]-bromid, trägerfrei, gegeben. Zu dieser Reaktionslösung fügt man 35 µg 0,125 µMol Chloramin-T (p-Toluolsulfon[N-chlor-N-natrium]-amid gelöst in 5 µl Wasser.

Nach 2 Stunden Reaktionsdauer, unter wiederholter DC-Kontrolle, gibt man die Reaktionslösung über eine Niederdrucksäule im System Hexan/Aceton (Aceton 0--- 30%). Man erhält etwa 74 MBq [$^{82}$Br] trägerfrei markiertes 17α-[$^{82}$Br]-bromethinyl-17β-hydroxy-18-methyl-4-östren-3-on. Die spezifische Aktivität liegt bei 80,5 TBq/mMol.

Wie für Beispiel 22 beschrieben kann die Substanz für diagnostische Zwecke sterilisiert werden.

Wie in Beispiel 22 und 23 beschrieben, lässt sich durch einen analogen Austausch mit einem radioaktiven Brom-Isotop, wie $^{77}$Br, $^{80m}$Br, $^{80}$Br, $^{82}$Br, die mit einem Brom-Isotop markierte Verbindung sowohl trägerfrei als auch geringerer spezifischen Aktivität herstellen. Die Reaktion wird durch Kupfersulfat katalysiert und gegebenenfalls zum Erzielen einer höheren Geschwindigkeitskonstante in Hexametapol® oder anderen hochsiedenden polaren Lösemitteln ausgeführt.

## Patentansprüche

1. Verfahren zur Herstellung von Brom und Jod unmarkierten und radioaktiv markierten 17α-Bromethinyl- und 17α-Jodethinyl-17β-hydroxysteroiden der allgemeinen Formeln I und II

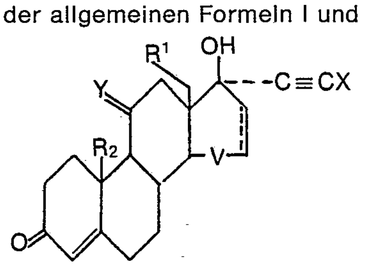

(I),

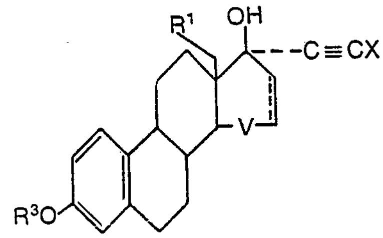

(II),

worin

..... eine Einfachbindung oder Doppelbindung ist,

V eine Kohlenstoff-Kohlenstoffbindung oder eine Methylengruppe darstellt,

X für ein Brom oder Jod steht,

Y zwei Wasserstoffatome oder eine Methylengruppe darstellt,

$R^1$ und $R^2$ ein Wasserstoffatom oder eine Methylgruppe bedeutet und

$R^3$ eine Alkylgruppe mit bis zu 4 Kohlenstoffatomen oder ein Wasserstoffatom darstellt aus entsprechenden 17α-Ethinyl-17β-hydroxysteroiden, dadurch gekennzeichnet, dass man das Ausgangssteroid in einem inerten Lösungsmittel mit einem Bromierungsmittel bzw. Jodierungsmittel in Gegenwart eines Silbersalzes behandelt.

2. 17α-Bromethinyl- oder 17α-Jodethinyl-17β-hydroxysteroide der allgemeinen Formel Ia

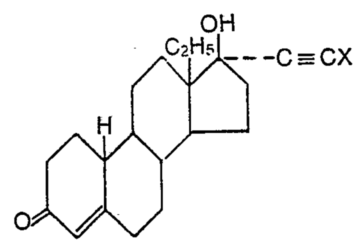

(Ia),

worin

X die im Anspruch 1 genannte Bedeutung besitzt.

## Claims

1. Process for the preparation of bromine and iodine unlabelled and radioactively labelled 17α-bromoethynyl- and 17α-iodoethynyl-17β-hydroxysteroides of the general formulae I and II

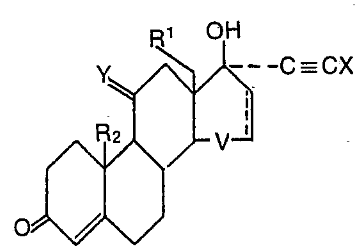

(I)

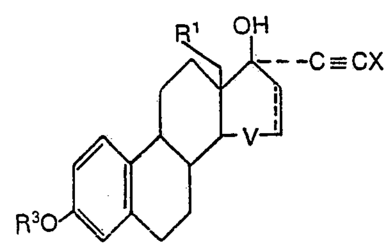

(II)

in which

..... is a single bond or a double bond,

V represents a carbon-carbon bond or a methylene group,

R represents hydrogen or methyl,

X represents bromine or iodine,

Y represents two hydrogen atoms or a methylene group,

$R^1$ and $R^2$ each represents a hydrogen atom or a methyl group, and

$R^3$ represents an alkyl group having up to 4 carbon atoms or a hydrogen atom,

from corresponding 17α-ethynyl-17β-hydroxysteroids, characterised in that the starting steroid is treated in an inert solvent with a brominating agent or an iodinating agent in the presence of a silver salt.

2. 17α-bromoethynyl- or 17α-iodoethynyl-17β-hydroxysteroids of the general formula Ia

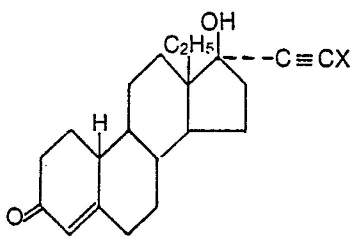

(Ia)

in which X has the meanings given in claim 1.

**Revendications**

1. Procédé pour préparer des bromethynyl-17α ou des iodéthynyl-17α stéroïdes marqués ou non marqués par du brome radioactif ou de l'iode radioactif, qui répondent aux formules générales I et II:

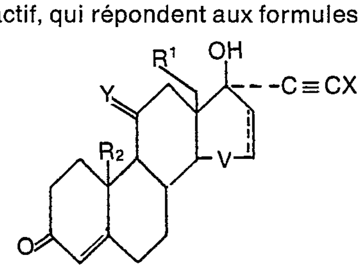

(I),

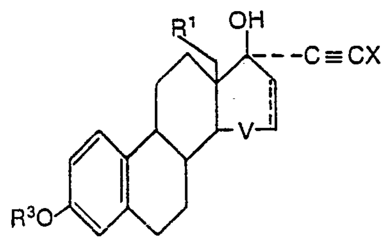

(II),

dans lesquelles:

..... représente une liaison simple ou une liaison double,

V représente une liaison carbone-carbone ou un radical méthylène,

X représente un atome de brome ou d'iode,

Y représente deux atomes d'hydrogène ou un radical méthylène,

$R^1$ et $R^2$ représentent chacun un atome d'hydrogène ou un radical méthyle et

$R^3$ représente un radical alkyle contenant au plus 4 atomes de carbone ou représente un atome d'hydrogène, à partir d'éthynyl-17α hydroxy-17β stéroïdes correspondants, procédé caractérisé en ce qu'on traite le stéroïde de départ, dans un solvant inerte, respectivement par un agent de bromation ou un agent d'iodation, en présence d'un sel d'argent.

2. Brométhynyl-17α ou iodéthynyl-17α hydroxy-17β stéroïdes qui répondent à la formule générale Ia:

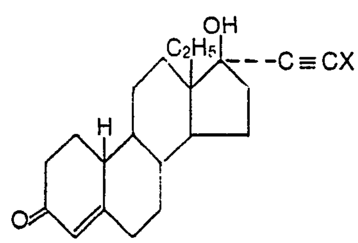

(Ia),

dans laquelle X a la signification qui lui a été donnée à la revendication 1.